# EUROPEAN PATENT APPLICATION

(11) **EP 3 228 247 A1**
(43) Date of publication of application: **11.10.2017**
(21) Application number: 16193847.7
(22) Date of filing: 14.10.2016
(51) Int. Cl.: A61B 5/083, A61B 5/097

(54) **BREATH TEST SYSTEM**

(30) Priority: 05.04.2016 JP 2016075811
(71) Applicant: Japan Precision Instruments Inc., Shibukawa-shi Gunma 377-0293 (JP)
(72) Inventor: Yoshida, Akira, Shibukawa-shi, Gunma 377-0293 (JP)
(74) Representative: Betten & Resch

(57) **Abstract**

There is provided a breath test system capable of further correctly performing a diagnosis of DOPD even if a carbon dioxide concentration in an artery is not measured, including: a breath test device; a device body 51 communicably connected to the breath test device 1, the breath test device 1 including: a flow path forming member having a flow path for flowing a respiratory gas of an examinee; a gas sensor having a light emitting section that emits an infrared ray to the flow path, and a light receiving section that receives an infrared ray emitted by the light emitting section; and a mouthpiece connected to the flow path forming member in a state of communicating with the flow path, the device body 51 including: a measuring section 52 that measures a carbon dioxide concentration contained in an exhaled breath of an examinee, based on an electric signal outputted from the gas sensor; an extracting section 60 that extracts a maximum value of the carbon dioxide concentration from a measurement result of the measuring section obtained in a period of N-times (N is an integer of 2 or more) breathing of the examinee; and a display section 57 for displaying a maximum value of the carbon dioxide concentration extracted by the extracting section 60.

## Description

### Technical Field

The present invention relates to a breath test system capable of measuring a carbon dioxide concentration contained in an exhaled breath.

### Description of Related Art

There is a chronic obstructive pulmonary disease (also referred to as "COPD" hereafter) as one of the respiratory diseases. When you develop COPD, symptoms such as shortness of breath and coughing, and sputum, etc., occur, and the symptoms will be severe with a progress of the disease. The diagnosis of COPD includes (1) a method for measuring a concentration of carbon dioxide in blood of the arterial blood, (2) a method using a spirometer (lung function test device), and (3) a method for measuring the concentration of carbon dioxide contained in the exhaled breath. Further, as a method for measuring the concentration of carbon dioxide in the exhaled breath, a method using an instrument called an airway adapter and a detector called a capnometer in combination, is known (for example, see patent documents 1 and 2).

Incidentally, the concentration of carbon dioxide (called a carbon dioxide concentration hereafter) in the blood of the arterial blood is represented by PaCO₂, and the carbon dioxide concentration contained in the exhaled breath is represented by EtCO₂ Among them, PaCO₂ is a term for an arterial carbon dioxide partial pressure, and EtCO₂ is a term for an end-tidal carbon dioxide concentration (end tidal CO₂).

### Prior Art Document

### Patent document

Patent document 1: Japanese Patent Laid Open Publication No.2012-159386
Patent document 2: Japanese Patent Laid Open Publication No.2014-160080

### Summary of the Invention

### Problem to be solved by the Invention

However, the following problem is involved in the abovementioned conventional techniques.

The method for measuring the carbon dioxide in arterial blood is capable of correctly performing a diagnosis of COPD, but on the other hand involves a problem that it is hardly widespread universally because blood is required to be collected from artery. Further, the method using a spirometer is simply the method for estimating COPD as one of a possibility of lung disease when a measured value of a lung capacity is outside a normal range, thus involving a problem that it is not suitable for a correct diagnosis of COPD. Further, the method using the airway adapter and the capnometer is the method of inserting an intubation tube into a patient's trachea from a mouth, with the airway adapter attached to the intubation tube. Therefore, the target of such a method is only the patients who are subjected to insertion of the intubation tube, (for example, the patient who needs to be mechanically ventilated). Thus, this method is not suitable for a correct diagnosis of COPD.

A main object of the present invention is to provide a technique of further correctly performing a diagnosis of COPD even if the carbon dioxide concentration in the artery is not measured.

### Means for solving the problem

### (First aspect)

A first aspect of the present invention is a breath test system, including:
a breath test device;
a device body communicably connected to the breath test device,
the breath test device including:
   a flow path forming member having a flow path for flowing a respiratory gas of an examinee;
   a gas sensor having a light emitting section that emits an infrared ray to the flow path, and a light receiving section that receives an infrared ray emitted by the light emitting section; and
   a mouthpiece connected to the flow path forming member in a state of communicating with the flow path,
the device body including:
   a measuring section that measures a carbon dioxide concentration contained in an exhaled breath of an examinee, based on an electric signal outputted from the gas sensor;
   an extracting section that extracts a maximum value of the carbon dioxide concentration from a measurement result of the measuring section obtained in a period of N-times (N is an integer of 2 or more) breathing of the examinee; and
   a display section for displaying a maximum value of the carbon dioxide concentration extracted by the extracting section.

### (Second aspect)

A second aspect of the present invention is the breath test system of the first or second aspect, wherein the extracting section includes:
a counting section that counts the number of breaths; and
a first detecting section that detects a maximum value of a carbon dioxide concentration per one breath of each time, based on a count result of the counting section; and
a second detecting section that detects a maximum value of a carbon dioxide concentration in a respiratory rate period of the N-times, by comparing maximum values of a carbon dioxide concentration detected by the detecting section in each time respiration, when the number of times counted by the counting section reaches the N-times,
wherein the maximum value of the carbon dioxide concentration detected by the second detecting section is displayed on the display section.

### (Third aspect)

A third aspect of the present invention is the breath test system of the first or second aspect, wherein the N is 3 or more and 10 or less.

### Advantage of the Invention

According to the present invention, diagnosis of COPD can be correctly performed even if a carbon dioxide concentration in artery is not measured.

### Brief description of the drawings

FIG. 1 is a schematic block diagram of a breath test system according to an embodiment of the present invention.
FIG. 2 is a side view of a breath test device according to an embodiment of the present invention.
FIG. 3 is a plan view of the breath test device according to an embodiment of the present invention.
FIG. 4A is a view illustrating the breath test device of FIG. 3 viewed from a left direction, and FIG. 4B is a view of the same viewed from a right direction.
FIG. 5 is an exploded perspective view of the breath test device according to an embodiment of the present invention.
FIG. 6 is a cross-sectional view illustrating a state before assembling a pair of case halves.
FIG. 7 is a cross-sectional view illustrating a state of assembling a pair of case halves.
FIG. 8A is a front view illustrating a configuration of an airway half, and FIG. 8B is a front view, and FIG. 8C is a rear view.
FIG. 9 is a cross-sectional view illustrating a state of mounting a sensor substrate on the airway half.
FIG. 10 is a cross-sectional view illustrating a state before assembling a pair of airway halves.
FIG. 11 is a cross-sectional view illustrating a state of assembling a pair of airway halves.
FIG. 12 is a schematic view illustrating a configuration example of a device body according to an embodiment of the present invention.
FIG. 13 is a view illustrating an example of an output waveform of a carbon dioxide concentration.

### Detailed description of the Invention

Embodiments of the present invention will be described hereafter, with reference to the drawings.

Embodiments of the present invention will be described in the following order.
1. Configuration of a breath test system
2. Configuration of a breath test device
3. Assembly of the breath test device
4. Configuration of a device body
5. Usage of the breath test device
6. Effect of an embodiment
7. Modified example, etc.

### <1. Configuration of a breath test system>

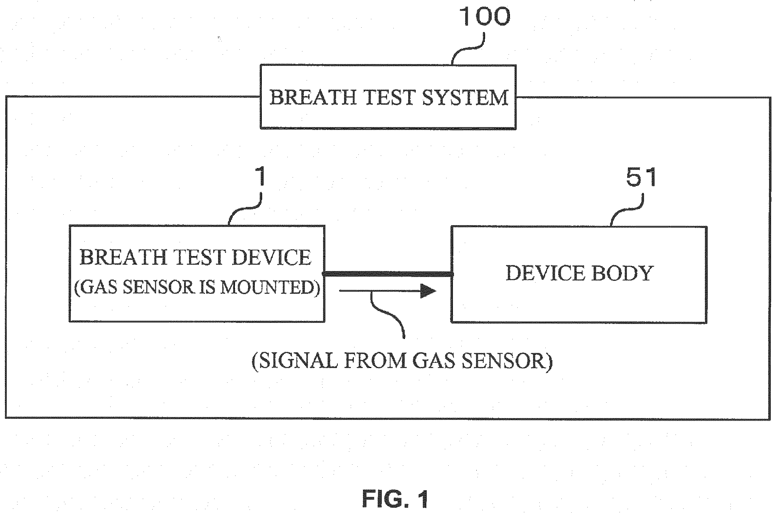

FIG. 1 is a schematic block diagram of a breath test system according to an embodiment of the present invention.

A breath test system 100 illustrated in the figure has a configuration roughly including a breath test device 1 and a device body 51. The breath test device 1 is handled by an examinee during a breath test, and the device body 51 is handled by a tester (such as a doctor or a nurse, etc.) during the breath test. The breath test device 1 and the device body 51 are communicably connected to each other. In this embodiment, as an example, the breath test device 1 and the device body 51 are connected by a cable.

### <2. Configuration of a breath test device>

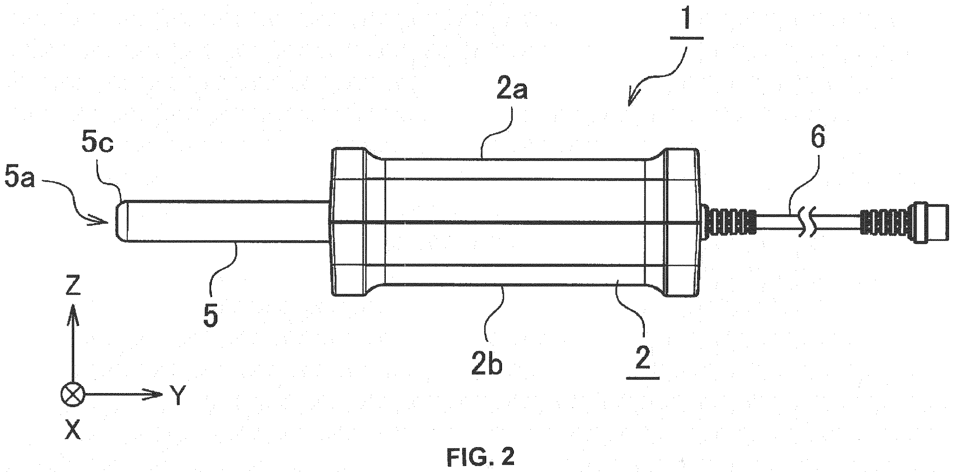

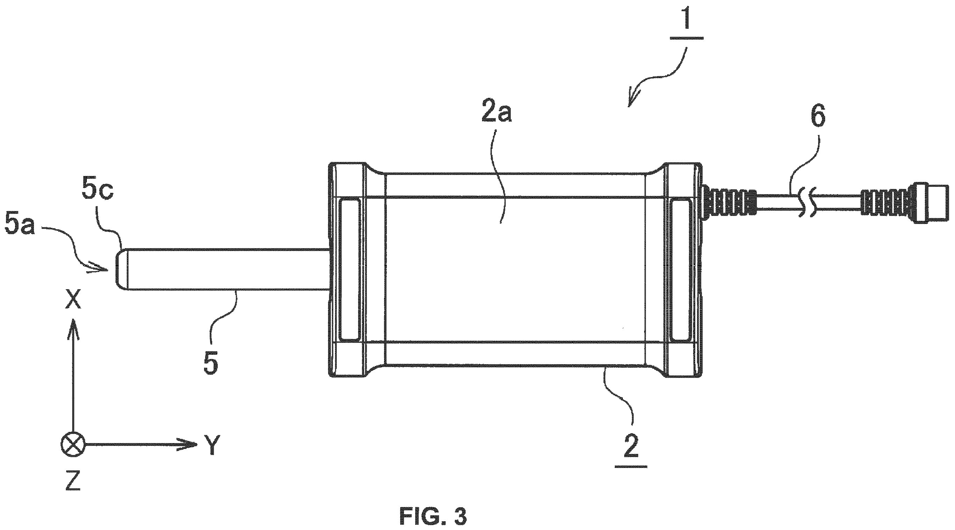

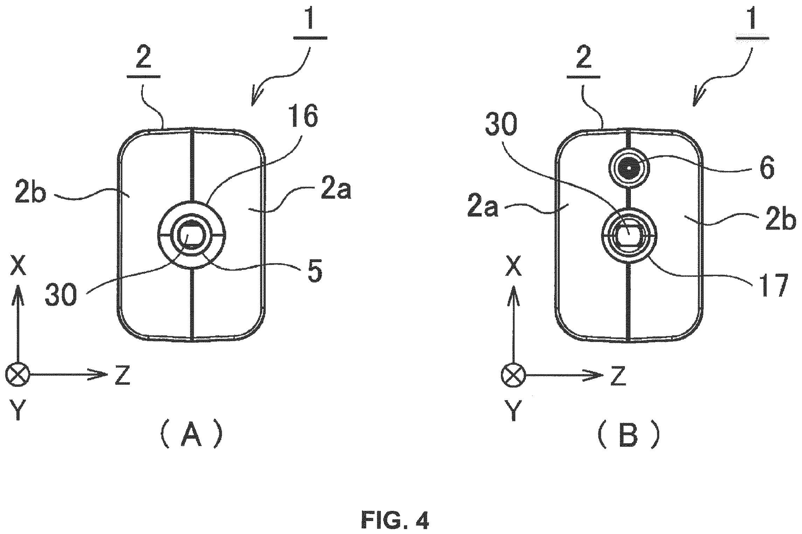

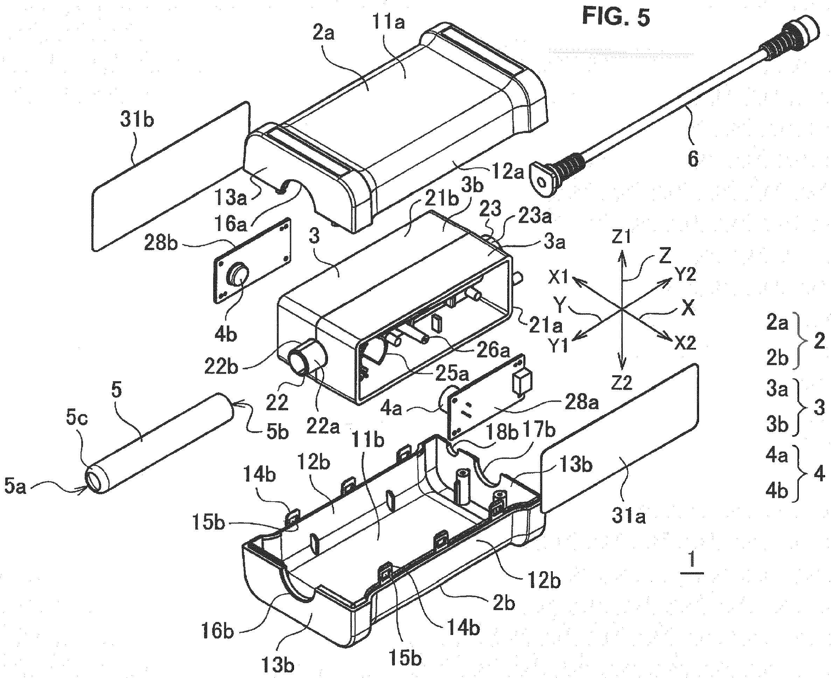

FIG. 2 is a side view of a breath test device according to an embodiment of the present invention, and FIG. 3 is a plan view of the same. Further, FIG. 4A is a view of the breath test device of FIG. 3 viewed from a left direction, and FIG. 4B is a view of the same viewed from a right direction. FIG. 5 is an exploded perspective view of the breath test device according to an embodiment of the present invention.

The breath test dev ice 1 illustrated in the figure has a configuration including a case 2 (2a, 2b), an airway member 3 (3a, 3b) as a flow path forming member, a gas sensor 4 (4a, 4b), a mouthpiece 5, and a cable 6.

In this embodiment, to describe the configuration and positional relation, etc., of each section of the breath test device 1, a width direction of the breath test device 1 is set as X-direction, a length direction of the breath test device 1 is set as Y-direction, and a height direction of the breath test device 1 is set as Z-direction. Further, as illustrated in FIG. 4, one of the X-directions is set as X1-direction, the other direction is set as X2-direction, and one of the Y-directions is set as Y1-direction, and the other direction is set as Y2-direction, and one of the Z-directions is set as Z1-direction, and the other direction is set as Z2-direction. Further, X1-direction is set as a left direction, X2-direction is set as a right direction, Y1-diretion is set as front direction, Y2-diretino is set as a depth direction, Z1-direction is set as an upper direction, and Z2-direction is set as a lower direction, based on a sight line when an examinee who receives a breath test views the breath test device 1 from the mouthpiece 5 side. However, the width direction and the height direction are exchanged, or the upper and lower, and right and left directions are exchanged in some cases, depending on the direction of the breath test device 1.

### (Case)

A case 2 has a half-split structure dividable into one and the other directions in Z-direction. The case 2 is configured by a pair of case halves 2a and 2b. In the pair of case halves 2a and 2b, a case half 2a constitutes an upper half of the case 2, and the other case half 2b constitutes a lower half of the case 2. Configurations of the case halves 2a and 2b will be described hereafter in further detail.

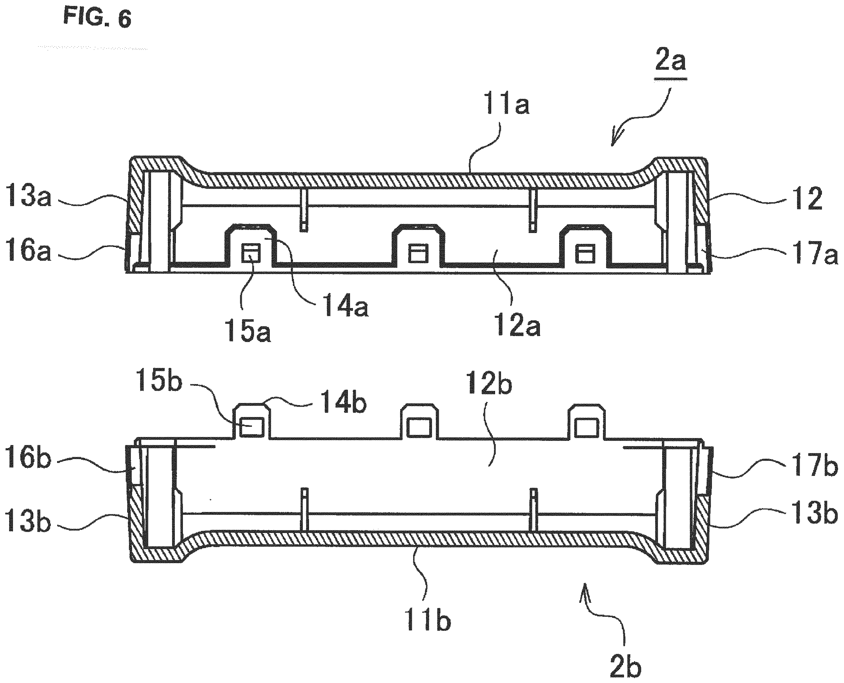

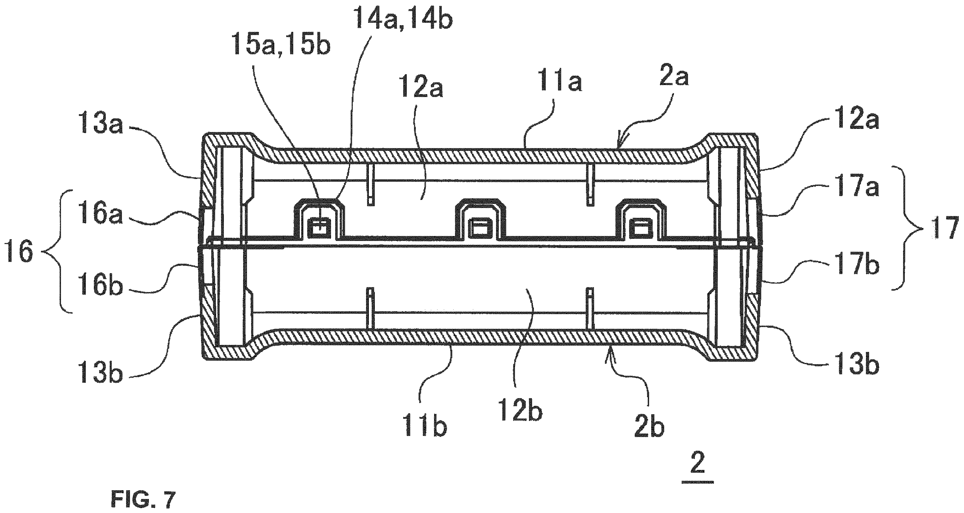

FIG. 6 is a cross-sectional view illustrating a state before assembling a pair of case halves, and FIG. 7 is a cross-sectional view illustrating a state of assembling the pair of case halves.

The case half 2a can be made of resin or metal, etc., but preferably may be made of resin, and more preferably made of ABS resin, in consideration of a cost, etc. The ABS resin is excellent in moldability, surface appearance, and impact resistance, etc., and therefore is preferable as a material of the case 2.

The case half 2a has a square (rectangular) main wall part 11a in plan view, a pair of side wall parts 12a facing each other in X-direction, and a pair of end wall parts 13a facing each other in Y-direction. The main wall part 11 a is a wall having a largest area among outer wall parts of the case half 2a. Locking parts 14a are respectively provided on the pair of side wall parts 12a. Three locking parts 14a are provided respectively on each inner surface of the side wall part 13a. The number and an arrangement of the locking parts 14a can be changed as needed. Each locking part 14a is formed in a state in which the inner surface of the side wall part 13a is partially recessed. A claw 15a is formed on each locking part 14a. The claw 15a is formed in a state of protruding to an inside of the case half 2a in X-direction. Further, a first notch 16a is formed on one of the end wall parts 13a, and a second notch 17a is formed on the other end wall part 13a. The first notch 16a is formed into a semicircular shape viewed from Y-direction, and the second notch 17a is also formed into a semicircular shape.

Similarly to the abovementioned case half 2a, the case half 2b has a square (rectangular) main wall part 11b in plan view, a pair of side wall parts 12b facing each other in X-direction, and a pair of end wall parts 13b facing each other in Y-direction. Locked parts 14b are provided respectively on the pair of side wall parts 12b. Three locked parts 14b are provided respectively on each upper edge of the side wall part 12b in a state of protruding in Z1-direction. The number and the arrangement of the locked parts 14b can be changed as needed. A hole 15b is formed on each locked part 14b, corresponding to the abovementioned claw 15a. The hole 15b is formed in a state of passing through the locked part 14b in a thickness direction. The claw 15a of the locking part 14a and the hole 15b of the locked part 14b corresponding to the claw 15a, are formed engageably with each other. Further, a first notch 16b is formed on one end wall part 13b, and a second notch 17b is formed on the other end wall part 13b. The first notch 16b is formed into a semicircular shape viewed from Y-direction, and the second notch 17b is also formed into the semicircular shape.

The pair of case halves 2a and 2b having the abovementioned configurations constitute the case 2 by assembling the case halves 2a and 2b in a state of aligning the positions of the mutually corresponding locking part 14a and the locked part 14b. In this configuration, the claw 15a of the locking part 14a is set in a state of engaging with the hole 15b of the locked part 14b corresponding to the claw 15a, and in this state, separation of the case halves 2a and 2b is inhibited, to thereby integrally form the case 2. The case 2 is a housing having a hollow part inside. Further, as illustrated in FIG. 6, first notches 16a and 16b constitute a first opening 16 in combination with each other, and second notches 17a and 17b constitute a second opening 17 in combination with each other. The first opening 16 and the second opening 17 are formed into a circular shape respectively.

### (Airway member)

The airway member 3 is configured to be dividable into half-split structure in X-direction as one direction and in the other direction. The airway member 3 is configured by a pair or airway halves 3a and 3b. In the pair of airway halves 3a and 3b, one airway half 3a forms a right half of the airway member 3, and the other airway half 3b forms a left half of the airway member 3. Configurations of the airway halves 3a and 3b will be described hereafter in further detail.

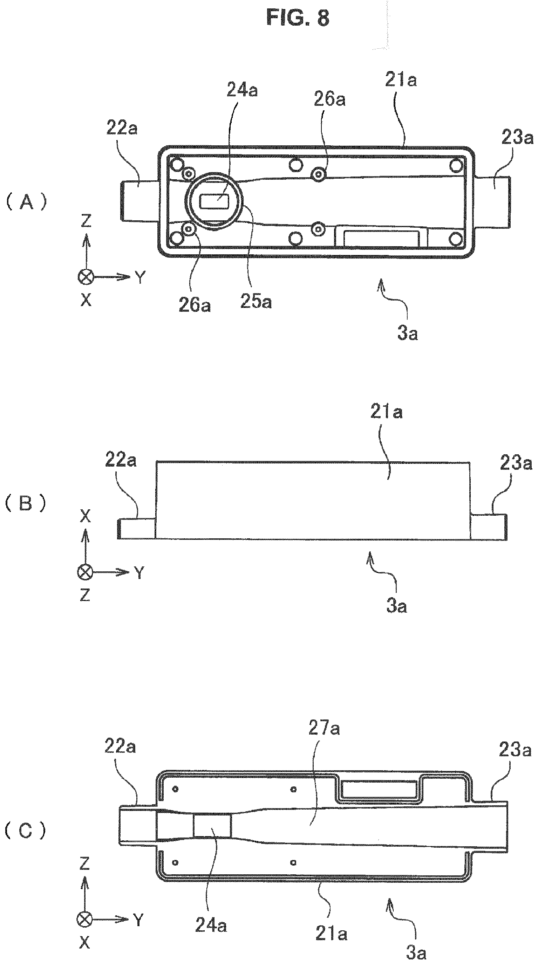

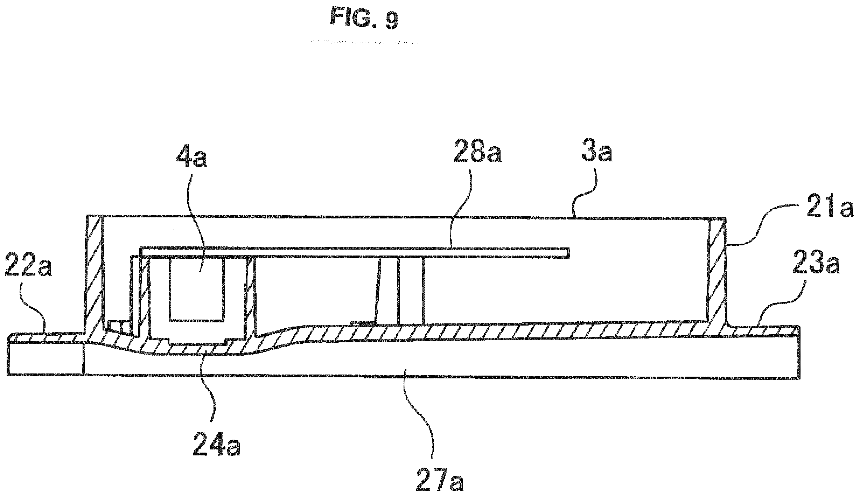

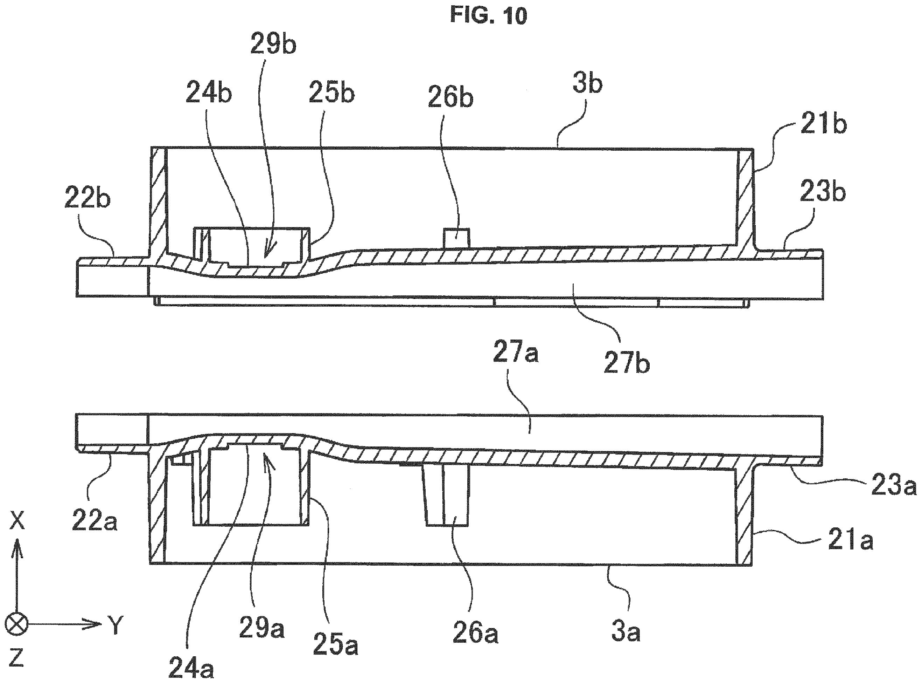

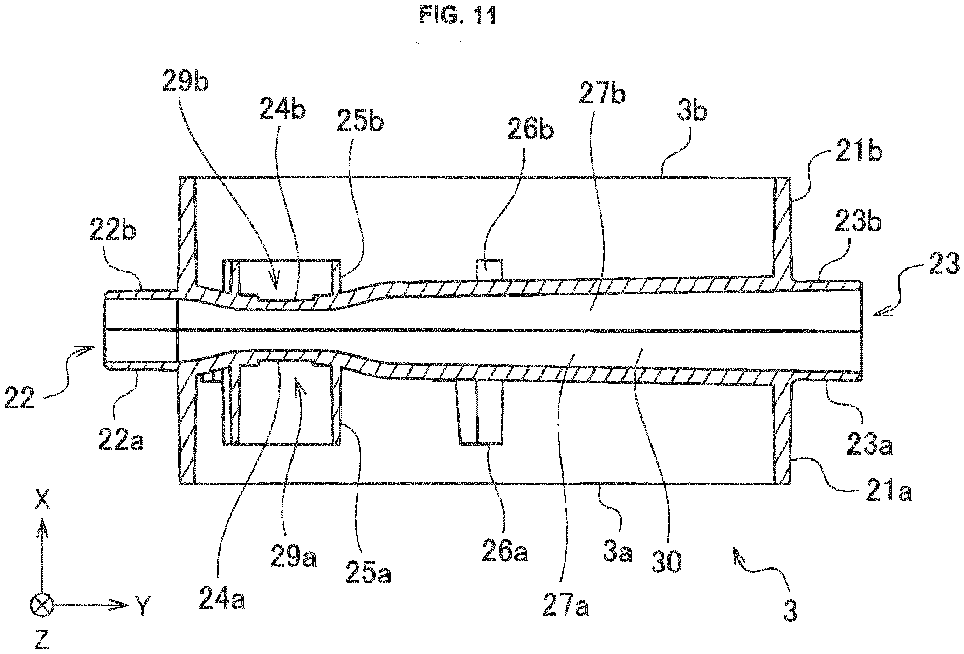

FIG. 8 illustrates the configuration of the airway half 3a, wherein FIG. 8A is a front view, FIG. 8B is a top view, and FIG. 8C is a rear view. Further, FIG. 9 is a cross-sectional view illustrating a state in which the first sensor substrate 28a is mounted on the airway half 3a. On the other hand, FIG. 10 is a cross-sectional view illustrating a state before assembling the pair of airway halves, and FIG. 11 is a cross-sectional view illustrating a state of assembling the pair of airway halves.

The pair of airway halves 3a and 3b has basically a similar configuration. Here, first, the configuration of one airway half 3a is described, and thereafter a difference between the configuration of the airway half 3a and the configuration of the airway half 3b will be described.

The airway half 3a integrally includes an outer frame 21a, a first semi-tubular portion 22a, a second semi-tubular portion 23a, a detection window 24a, a supporting frame 25a, a plurality of studs 26a, and a long groove 27a. Preferably the airway half 3a is integrally made of polyethylene terephthalate (also referred to as "PET" hereafter) having a transparent hue. "Integrally made of" described here, refers to a configuration in which each part of the airway half 3a is made of the same resin material (PET), and each part is continuously (seamlessly) connected. Such a configuration is obtained by integral molding (for example, injection molding, etc.). Incidentally, a previously molded small component (for example, injection molding) is set in a die for injection-molding, and a product obtained by injection-molding in this state inevitably has a seam on an outer shape portion of the small component (not seamless), and therefore is not included in the "integrally made of" called here.

The outer frame 21a is formed into a horizontally long rectangular shape viewed from X-direction. The first semi-tubular portion 22a is formed in a state of protruding from one end of the outer frame 21 a in a longitudinal direction (Y-direction), and the second semi-tubular portion 23a is formed in a state of protruding from the other end of the outer frame 21 a in the longitudinal direction. The first semi-tubular portion 22a and the second semi-tubular portion 23a are respectively formed into a half cylindrical shape viewed from Y-direction.

When a prescribed gas concentration contained in the exhaled breath is measured using the infrared ray, the detection window 24a is a window translucent to transmit the infrared ray. The detection window 24a is formed into a rectangular shape similarly to the outer frame 21a, viewed from X-direction. The detection window 24a is provided close to the semi-tubular portion 22a in Y-direction. The supporting frame 25a is formed for attaching the first sensor substrate 28a to the airway half 3a without positional interference with the light emitting section 4a. The supporting frame 25a is formed into approximately a circular shape along an outer periphery of the light emitting section 4a. The studs 26a are intended to attach the first sensor substrate 28a to the airway half 3a by screw cramping, and have a small hole on a central axis.

The long groove 27a is intended to form one flow path 30 when a pair of airway halves 3 a and 3b are assembled. The long groove 27a is formed into a long shape in Y-direction, so as to connect the first semi-tubular portion 22a and the second semi-tubular portion 23a. A groove width of the long groove 27a becomes partially narrow at the detection window 24a. Further, the groove width of the long groove 27a is formed wider gradually from the formation site of the detection window 24a toward the formation site of the second semi-tubular portion 23a.

Further, the thickness of the portion of the detection window 24a becomes smaller than the other portion that forms the long groove 27a. Specifically, the recess portion 29a is formed in a recessed state on the flow path 30 side, on a side opposite to the side facing the flow path 30, which is the formation site of the detection window 24a, and the portion of the detection window 24a becomes smaller than the other portion, due to a dent of the recess portion 29a. A thickness dimension of the portion of the detection window 24a is preferably set to 0.1 mm or more and 0.5 mm or less, and more preferably 0.2 mm or more and 0.4 mm or less (for example, 0.3 mm).

On the other hand, as illustrated in FIG. 10, the airway half 3b integrally includes an outer frame 21b, a first semi-tubular portion 22b, a second semi-tubular portion 23b, a detection window 24b, a supporting frame 25b, a plurality of studs 26b, and a long groove 27b. This point is also applied to the abovementioned airway half 3a. However, protruding dimensions of the supporting frames 25a, 25b and the studs 26a and 26b are different between the airway half 3a and the airway half 3b. In other words, the protruding dimension of the supporting frame 25a, etc., of the airway half 3a is set to be larger than the protruding dimension of the supporting frame 25b, etc., of the airway half 3b. This is because the height dimension of the light emitting section 4a mounted on the first sensor substrate 28a and the height dimension of the light receiving section 4b mounted on the second sensor substrate 28b, are different from each other. Specifically, the height dimension of the light emitting section 4a is set to be larger than the height dimension of the light receiving section 4b, and the protruding dimensions of the supporting frames 25a, 25b and the studs 26a, 26b are set according to the difference between the above dimensions.

The airway member 3 having the flow path 30 inside is constituted by assembling the airway halves 3a and 3b, in a state in which mutually corresponding portions of the airway halves 3a and 3b of abovementioned configurations are faced each other. The flow path 30 is formed by the long grooves 27a and 27b. The flow path 30 is formed so as to extend long in Y-direction. When the flow path 30 is viewed from Y-direction, the portion of the detection windows 24a and 24b is formed into a quadrangle (preferably rectangle or square), and the other portion is formed into a circular shape or a shape close to the circular shape.

A first tubular portion 22 is formed by the first semi-tubular portions 22a and 22b at a front side of the airway member 3, and a second tubular potion 23 is formed by the second semi-tubular portions 23a and 23b at a back side of the airway member 3. The first tubular portion 22 is formed in a state of protruding in Y1-direction from one of the end faces of the airway member 3 (outer frames 21a and 21b), and the second tubular portion 23 is formed in a state of protruding in Y-2 direction from the other end face of the airway member 3. The first tubular portion 22 is formed into a cylindrical shape, and the second tubular portion 23 is formed into a cylindrical shape with a larger diameter than the diameter of the first tubular portion 22. The first tubular portion 22 and the second tubular portion 23 are arranged concentrically with the flow path 30 in a state of communicating with the flow path 30 respectively.

Further, an airtight structure by engagement of irregularities, is employed on an abutting surface of the airway halves 3a and 3b. Specifically, although not illustrated, recess-shaped grooves are formed on the abutting surface of the airway half 3a, and protrusion-shaped ribs are formed on the abutting surface of the airway half 3b. Then, by an engagement of the ribs and the grooves, the airtightness of a space is maintained, the space extending to the second tubular portion 23 from the first tubular portion 22 through the flow path 30.

### (Gas sensor)

The gas sensor 4 is configured using the light emitting section 4a for emitting the infrared ray, and the light receiving section 4b for receiving the infrared ray. In this embodiment, the airway member 3 and the gas sensor 4 are integrally constituted by mounting the gas sensor 4 on the airway member 3. "Integrally constituted" described here, refers to a configuration in which the airway member 3 and the gas sensor 4 are fixed to each other utilizing a physical coupling unit (screw fastening or bonding, etc.), which are held together as one as a whole. Accordingly, for example as disclosed in Patent Laid Open Publication No.2012-159386 and Patent Laid Open Publication No.2014-160080, an embodiment in which the flow path forming member and the gas sensor are detachably constituted as a separate body, is not included in the abovementioned concept of "integrally constituted".

The light emitting section 4a is mounted on the first sensor substrate 28a, and the light receiving section 4b is mounted on the second sensor substrate 28b. The light emitting section 4a and the light receiving section 4b are arranged in a state of facing each other, with the flow path 30 of the airway member 3 interposed between them. Then, the light emitting section 4a emits the infrared ray toward the flow path 30, and the light receiving section 4b receives the infrared ray through the flow path 30.

### (Mouthpiece)

The mouthpiece 5 is constituted by a tubular member. The mouthpiece 5 is formed into a straw shape with longitudinal (Y-directional) both ends opened. The "straw shape" described here refers to a cylindrical shape with a central axis extending straight (straight line). Openings 5a and 5b are respectively formed at a front side and at a back side of the mouthpiece 5. A front side end portion of the mouthpiece 5 is a tapered portion 5c rounded inward, and the front side opening 5a has a smaller diameter than the diameter of the back side opening 5b, by a portion of the opening diameter reduced by the tapered portion 5c. Thus, by forming the tapered portion 5c at the end portion of the mouthpiece 5, the mouthpiece becomes mild (aggregable) to the teeth of the examinee owing to the rounded tapered portion 5c, when the opening 5a side of the mouthpiece 5 is held between the examinee's teeth. Further, when the examinee is breathing with the mouthpiece 5 held between teeth, the saliva is less likely to penetrate into the mouthpiece 5. The mouthpiece 5 may be made of any one of metal, resin, or paper.

The mouthpiece 5 is preferably disposable (throw-away product). In this case, the mouthpiece 5 is preferably made of resin or paper from a viewpoint of a cost, etc. In this embodiment, the mouthpiece 5 is an integral structure of resin. In this case, polypropylene is preferable as the material of the mouthpiece 5 from a viewpoint of easiness of manufacture and the cost, etc. The mouthpiece 5 is preferably provided in individual sterile packaging of several units to tens of several units, and more preferably in a separate sterile packaging of one unit, and it is preferable to use it by taking out from there one by one.

### (Cable)

The cable 6 is the cable for electrically connecting the breath test device 1 and a measuring device not illustrated. A length of the cable 6 is set to a suitable length as needed. The cable 6 incorporates a lead wire (not illustrated), and electrically connected to the first sensor substrate 28a and the second sensor substrate 28b, using the lead wire, etc. The cable 6 is used for suppling a power to the breath test device 1 from the measuring device for driving the gas sensor 4, transferring a control signal between the measuring device and the breath test device 1 for controlling a drive of the gas sensor 4, and outputting a result detected by the gas sensor 4 as an electric signal.

### <3. Assembly of the breath test device>

The breath test device 1 is assembled for example as follows.

First, a pair of airway halves 3a and 3b are assembled each other. At this time, the first sensor substrate 28a is mounted on one airway half 3a, and the second sensor substrate 28b is mounted on the other airway half 3b. Further, the light emitting section 4a is previously mounted on the first sensor substrate 28a, and the light receiving section 4b is previously mounted on the second sensor substrate 28b. The first sensor substrate 28a is fixed to the airway half 3a using a screw not illustrated. Specifically, four attachment holes are provided on the first sensor substrate 28a, and by screwing the screw into four studs 26a respectively through each attachment hole, the first sensor substrate 28a is fixed to the airway half 3a. Similarly, the second sensor substrate 28b is fixed to the airway half 3b using the screw not illustrated. At this time, the light emitting section 4a is housed in the supporting frame 25a of the airway half 3a, and the light receiving section 4b is housed in the supporting frame 25b of the airway half 3b.

Further, a protection sheet 31a is attached to the airway half 3a, and a protection sheet 31b is attached to the airway half 3b. At this time, before attaching each protection sheet 31a, 31b, the cable 6 and each sensor substrate 28a, 28b are electrically connected by the lead wire, etc., not illustrated. Such a connection is performed, for example, by an insertion of connectors in a relationship of male and female. The protection sheets 31a and 31b may be made of transparent PET for example similarly to the airway member 3. The protection sheet 31a is intended to achieve functions such as holding air tightness and dustproof of an internal space (mounting region of the light emitting section 4a and the first sensor substrate 28a) of the airway half 3a surrounded by the outer frame 21a, and the protection sheet 31b is also intended to have the same function. The protection sheet 31a is stuck to an opening edge of the outer frame 21 a using a double-sided tape or an adhesive, etc., so as to close a square opening of the outer frame 21a. The protection sheet 31b is also stuck to the opening edge of the outer fame 21b.

The airway member 3 thus assembled is for example set in a lower side case half 2b. At this time, the cable 6 is set in a notch 18b (FIG. 5) for attaching the cable provided to the case half 2b. Although not illustrated, the notch for attaching the cable, is also provided to the case half 2a.

Thereafter, a pair of case halves 2a and 2b are assembled each other so as to cover the case half 2b by the case half 2a. In this case, connection of the case halves 2a and 2b is achieved by the engagement between the claw 15a of the locking part 14a and the hole 14b of the locked part 14b. However, the present invention is not limited thereto, and both case halves may be connected (fixed) by screwing, etc. Further, a dividing direction of the case 2 constituted using a pair of case halves 2a and 2b, and a dividing direction of the airway member 3 constituted using a pair of airway halves 3a and 3b, are different directions from each other, and specifically in a relation in which the phase is mutually shifted by 90 degrees around the central axis of the flow path 30. In other words, the dividing direction of the airway member 3 is the X-direction, and the dividing direction of the case 2 is the Z-direction, in a direction perpendicular to a length direction (Y-direction) of the flow path 30.

Further, the opening edge of the first tubular portion 22 of the airway member 3 is arranged concentrically with the first opening 16 of the case 2, and the opening edge of the second tubular portion 23 of the airway member 3 is arranged concentrically with the second opening 17 of the case 2. In this state, the opening 5b side of the mouthpiece 5 is inserted and connected into/to the first tubular portion 22 of the airway member 3. Accordingly, the space inside of the mouthpiece 5 is set in a state of communicating with the flow path 30 of the airway member 3 through the first tubular portion 22.

Thus, with the mouthpiece 5 attached, a series of airway is formed from the opening 5a of the mouthpiece 5 to the first tubular portion 22 and the opening of the second tubular portion 23 through the flow path 30. Then, a pair of detection windows 24a and 24b are arranged in the middle of the airway, and preferably in a longitudinally (Y-directional) intermediate portion of the airway. Further, when a distance from the pair of detection windows 24a and 24b to the opening of the second tubular potion 23 is set to be too long in the length direction of the airway, air resistance becomes large, thus making it difficult to breathe.

As described above, in the breath test device 1 of this embodiment, the case 2 is constituted in a state of mutually assembling the pair of case halves 2a and 2b. The case 2 has a housing structure having a space inside. The airway member 3 is housed in the internal space of this case 2. Further, the case 2 holds the airway member 3 in the internal space so as not to be moved.

The airway member 3 includes the flow path 30 for flowing a respiratory gas (expiratory and inspiratory) of the examinee, the first tubular portion 22 connected to the flow path 30, and the second tubular portion 23 connected to the flow path 30 on the opposite side to the first tubular portion 22. The first tubular portion 22 and the second tubular portion 23 are formed into a cylindrical shape respectively. An inner diameter of the first tubular portion 22 is set to be larger than the inner diameter of the second tubular portion 23. Exhalation (exhaled breath) and inhalation (inhaled breath) of the examinee during breathing, flow in the flow path 30, through the first tubular portion 22 and the second tubular portion 23. In this case, the first tubular portion 22 is the portion for guiding the gas exhaled by the examinee to the flow path 30, and meanwhile discharging the gas inhaled by the examinee toward an oral cavity of the examinee. On the other hand, the second tubular portion 23 is the portion for discharging the gas exhaled by the examinee into the atmosphere, and meanwhile guiding the gas inhaled by the examinee into the flow path 30 from the atmosphere.

### <Configuration of a device body>

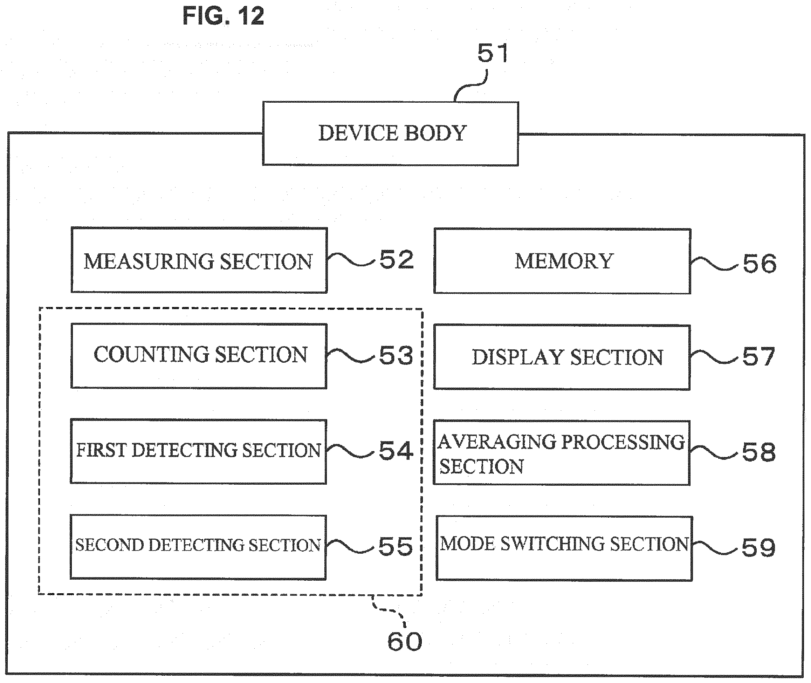

FIG. 12 is a schematic view illustrating a configuration example of a device body according to an embodiment of the present invention.

A device body 51 illustrated in the figure has a configuration including a measuring section 52, a counting section 53, a first detecting section 54, a second detecting section 55, a memory 56, a display section 57, an averaging processing section 58, and a mode switching section 59. Among them, the counting section 53, the first detecting section 54, and the second detecting section 55 constitute an extracting section 60. The extracting section 60 extracts a maximum value of a carbon dioxide concentration from a measurement result of the measuring section 52 obtained in a period of N-times (N is integer of 2 or more) breathing of the examinee. The value of N which is one of the conditions of extracting the maximum value of the carbon dioxide concentration, is preferably 3 or more and 10 or less, more preferably 4 or more and 8 or less, and further preferably 5 or more and 7 or less. In this embodiment, explanation is given for a case in which the value of N is set to N = 6, as a particularly preferable example.

### (Measuring section)

The measuring section 52 measures the carbon dioxide concentration (EtCo₂) contained in the exhaled breath of the examinee, based on the electric signal outputted from the gas sensor 4. In the measuring section 52, by applying a signal processing to the electric signal outputted from the gas sensor 4 based on a prescribed algorithm, the electric signal is converted to a numerical value (unit: mmHg) showing the carbon dioxide concentration in the exhaled breath. The value of the carbon dioxide concentration measured by the measuring section 52 is stored in the memory 56.

### (Counting section)

The counting section 53 counts the number of respiratory rates. In the counting section 53, the respiratory rate is counted using a waveform showing a time-varying change in a level of the electric signal outputted from the gas sensor 4, or a waveform showing a time-varying change in the carbon dioxide concentration (EtCo₂) measured by the measuring section 52. In this embodiment, as an example, the respiratory rate is counted using an output waveform of the carbon dioxide concentration, as an example. In this case, one respiratory period (called "one breathing period" hereafter) includes a period for exhaling breath and a period for inhaling breath, and the output waveform of the carbon dioxide concentration is changed in accordance with each period. Therefore, the counting section 53 counts the respiratory rate based on the change of the output waveform.

### (Detecting section)

The first detecting section 54 detects the maximum value of the carbon dioxide concentration per one breath of each time, based on a count result of the counting section 53. The first detecting section 54 detects a peak value where the carbon dioxide concentration is highest in the output waveform of the carbon dioxide concentration that appears in a mountain shape in every one breathing period, as the maximum value of the carbon dioxide per one breathing. The value of the carbon dioxide concentration measured by the measuring section 52 in the respiratory period of each time is stored in the memory 56, and therefore the first detecting section 54 detects the maximum value of the carbon dioxide concentration measured by the measuring section 52 in one breathing period, every time the respiratory rate is counted-up by one by the counting section 53. Therefore, the maximum value of the carbon dioxide concentration per one breath may be read as the maximum value of the carbon dioxide concentration in one breathing period. Further, when the maximum value of the carbon dioxide concentration per one breathing of each time is detected, the first detecting section 54 stores the detected maximum value of the carbon dioxide concentration in the memory 56, every time the detection is performed. Thus, the maximum value of the carbon dioxide concentration per one breathing period is stored in the memory 56.

### (Detecting section)

When the number of times of count by the counting section 53 reaches N-times (six times in this embodiment), the second detecting section 55 detects the maximum value of the carbon dioxide concentration in the breathing numbers of times period of N-times, by comparing the maximum values of the carbon dioxide concentration detected by the first detecting section 54 in the breathing of each time. When the value of N is set to N = 6 and after the respiratory rate counted by the counting section 53 reaches six, the second detecting section 55 compares a large/small relation of the maximum values of six carbon dioxide concentration stored in the memory 56 in accordance with six respiratory rates, and detects the maximum value of the carbon dioxide concentration which is a largest among them.

### (Display section)

The display section displays a measurement state or a measurement result of the breath test. The measurement result includes the maximum value of the carbon dioxide concentration extracted by the extracting section 60. Further, preferably the measurement result includes the output waveform of the carbon dioxide concentration. The maximum value of the carbon dioxide concentration extracted by the extracting section 60 may be displayed in such manner that it is always included in the measurement result of the respiratory rate, or may be displayed by switching of a processing mode by the mode switching section 59 described later. In this embodiment, the extracting section 60 is constituted by the abovementioned counting section 53, the first detecting section 54, and the second detecting section 55, and therefore the maximum value of the carbon dioxide concentration detected by the second detecting section 55 is displayed on the display section 57. The display section 57 may be constituted using, for example, a liquid crystal display and an organic EL display, etc.

### (Averaging processing section)

The averaging processing section 58 applies averaging processing to the maximum value of the carbon dioxide concentration detected by the first detecting section 54. In this embodiment, as an example, a moving average method is used. In this case, the averaging processing section 58 averages the maximum value of the carbon dioxide concentration detected by the first detecting section 54 using the moving average method, every time the respiratory rate counted by the counting section 53 is increased by one. For example, at a time point of five respiratory rates counted by the counting section 53, five maximum values of the carbon dioxide concentration detected by the first detecting section 54 are averaged. The averaging processing may be finished in a stage when the respiratory rate counted by the counting section 53 reaches N-times (six in this embodiment), or may be continued exceeding N-times.

### (Mode switching section)

The mode switching section 59 switches the processing mode of the device body 51. The processing mode of the device body 51 includes at least two processing modes. One of them is a first processing mode that operates so that the maximum value of the carbon dioxide concentration is displayed on the display section 57, the maximum value in this case being extracted by the extracting section 60, and the other one is a second processing mode that operates so that the maximum value of the carbon dioxide concentration is displayed on the display section 57, the maximum value in this case being subjected to averaging processing by the averaging processing section 58. When the device body 51 is operated in each processing mode, the output waveform of the carbon dioxide concentration measured by the measuring section 52 is preferably displayed on the display section 57.

### <5. Usage of the breath test device>

The usage of the breath test device 1 of this embodiment will be described next.

First, the breath test device 1 with the mouthpiece 5 is held by a hand of the examinee who receives a breath test. Next, the opening 5a side of the mouthpiece 5 is held between the teeth of the examinee. Next, the examinee is asked to perform spontaneous breathing, and in this state, the breath test is carried out using the gas sensor 4. In this breath test, concentration of the carbon dioxide (EtCO₂) in the exhaled breath is measured as an example of the prescribed gas concentration contained in the exhalation of the examinee. In the measurement of the concentration of the carbon dioxide, for example the light emitting section 4a may be used, which is configured to emit an infrared ray with a wavelength of 3.75 µm or more and 4.25 µm or less.

When the examinee performs breathing with the mouthpiece 5 in the mouth, the exhaled breath or the inhaled breath alternately flows through the flow path 30 of the breath test device 1, in accordance with the breathing of the examinee. At this time, a moving direction of the exhaled breath flowing through the flow path 30 when the examinee breathe out, and a moving direction of the inhaled breath flowing through the flow path 30 when the examinee breathe in, are opposite directions to each other. In other words, when the examinee breathe out, the exhaled breath flows through the flow path 30 from the first tubular portion 22 toward the second tubular portion 23, and when the examinee breathe in, the inhaled breath flows through the flow path 30 from the second tubular portion 23 toward the first tubular portion 22.

Under such a circumstance, the gas sensor 4 emits the infrared ray from the light emitting section 4a and receives the infrared ray by the light receiving section 4b, to thereby detect the concentration of the carbon dioxide in the breath that flows through the flow path 30. At this time, the infrared ray emitted by the light emitting section 4a transmits the detection window 24a of the airway half 3a and advances to the flow path 30. Further, the infrared ray that advances to the flow path 30, passes across the flow path 30, and thereafter transmits the detection window 24b of the airway half 3b and reaches the light receiving section 4b.

On the other hand, the carbon dioxide has a property of absorbing the infrared ray emitted from the light emitting section 4a. Therefore, when the concentration of the carbon dioxide flowing through the flow path 30 is relatively high, the ratio of the infrared ray absorbed by the carbon dioxide becomes large accordingly. Therefore, a light reception amount of the infrared ray by the light receiving section 4b becomes relatively small. Reversely, when the concentration of the carbon dioxide flowing through the flow path 30 is relatively low, the ratio of the infrared ray absorbed by the carbon dioxide becomes small accordingly. Therefore, the light reception amount of the infrared ray by the light receiving section 4b becomes relatively large. Accordingly, the concentration of the carbon dioxide contained in the exhaled breath of the examinee can be measured based on the light reception amount of the infrared ray received by the light receiving section 4b.

In an actual measurement, an electric signal outputted from the light receiving section 4b is transmitted to a device body 51 through the cable 6. At this time, in the device body 51, the following processing is performed. Here, as an example, explanation is given for a case in which the processing mode of the device body 51 is set to the first processing mode by the mode switching section 59.

First, the measuring section 52 measures the carbon dioxide concentration in the exhaled breath, based on the electric signal outputted from the gas sensor 4. Specifically, the measuring section 52 converts the electric signal to a numerical value (unit: mmHg) showing the carbon dioxide concentration (EtCo₂) in the exhaled breath, by processing the electric signal outputted from the gas sensor 4 in accordance with a prescribed algorithm. Further, the measuring section 52 stores the measured value of the carbon dioxide concentration in the exhaled breath, in the memory 56, and creates the output waveform of the carbon dioxide concentration showing the measurement result, and displays it on the display section 57.

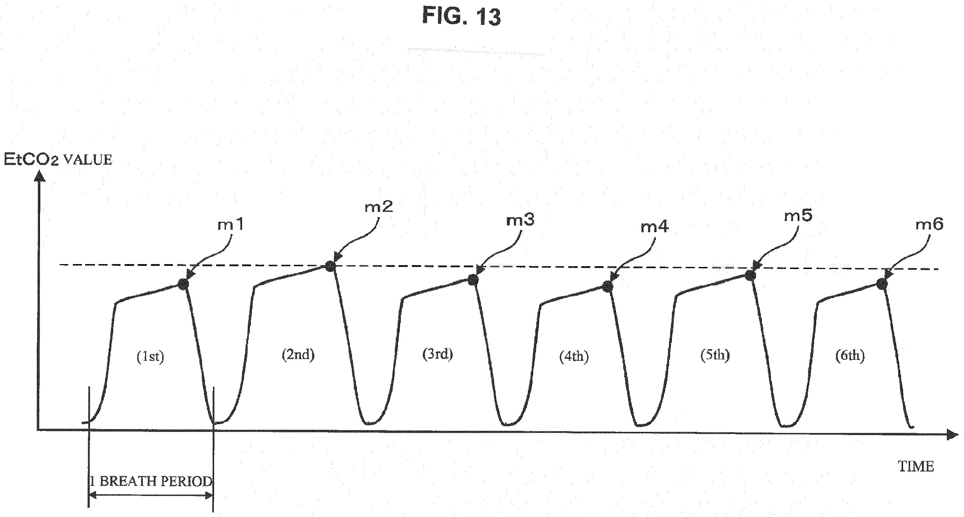

Further, the counting section 53 counts the respiratory rate of the examinee, using the output waveform of the carbon dioxide concentration. FIG. 13 illustrates an example of the output waveform of the carbon dioxide concentration. In FIG. 13, the vertical axis indicates a measurement value of the carbon dioxide concentration (EtCO₂), and the horizontal axis indicates time. When the examinee actually repeats breathing, the output waveform of the carbon dioxide concentration is changed as follows. In other words, when the examinee starts to exhale the breath, the value of the carbon dioxide concentration rises sharply, and thereafter turns to a gradual upward trend. Subsequently, when the examinee starts to inhale the breath, the value of the carbon dioxide concentration is decreased sharply. Therefore, the output waveform of the carbon dioxide concentration is a mountain-shaped waveform, and such a mountain-shaped waveform appears continuously in accordance with the respiratory rate of the examinee.

Further, in a state before starting breathing by the examinee, the measured value of the carbon dioxide concentration based on the electric signal from the gas sensor 4 is 0 mmHg or the value close thereto (several mmHg). Therefore, the counting section 53 detects a timing when the carbon dioxide concentration measured by the measuring section 52 exceeds a prescribed value (for example, 20 mmHg) as an expiration start timing, and thereafter detects a timing when the carbon dioxide concentration is below the prescribed value as an intake end timing. Then, a period from the expiration start timing to the intake end timing is regarded as the "one breathing timing", and this one breathing timing (in other words, one mountain-shaped waveform) is counted as one breathing. Thus, the respiratory rate counted by the counting section 53 is added by one, every time the examinee performs one breathing.

On the other hand, when the respiratory rate counted by the counting section 53 is added by one, the first detecting section 54 detects the maximum value of the carbon dioxide concentration per one breathing period. For example, when the respiratory rate counted by the counting section 53 is one, maximum value m1 of the carbon dioxide concentration in one breathing is detected, based on the measurement result of the carbon dioxide concentration obtained by the measuring section 52 in the first breathing. Further, when the respiratory rate counted by the counting section 53 is two, maximum value m2 of the carbon dioxide concentration in one breathing period is detected, based on the measurement result of the carbon dioxide concentration obtained by the measuring section 52 in the second breathing. Similarly in the third breathing or the breathing thereafter, maximum values m3, m4, m5, and m6 of the carbon dioxide concentration are sequentially detected. Thus, the maximum value of the carbon dioxide concentration per one breathing in each time of the respiratory rate counted by the counting section 53, is detected by the first detecting section 54, and the detection result is stored in the memory 56.

Thereafter, when the respiratory rate counted by the counting section 53 reaches six, the second detecting section 55 detects the maximum value of the carbon dioxide concentration in the six respiratory rate periods. Specifically, the first detecting section 54 compares the large/small relation of the six maximum values m1 to m6 of the carbon dioxide concentration stored in the memory 56 in accordance with the six respiratory rates, and detects the maximum value of the carbon dioxide concentration which is the largest value of the maximum values m1 to m6. In the output waveform of the carbon dioxide concentration illustrated in FIG. 13, the maximum value m2 of the carbon dioxide concentration detected by the first detecting section 54 in the second breathing is the largest value, and therefore the second detecting section 55 detects the maxima value m2 of the carbon dioxide concentration.

The maximum value of the carbon dioxide concentration thus detected by the second detecting section 55 is displayed on the display section 57. In this case, in a period until finish of the six breathings by the examinee, a number indicating the respiratory rate of the examinee may be displayed on the display section 57 in a countdown format such as "6" → "5" → "4" → (omitted) → "1", or the maximum value of the carbon dioxide concentration detected by the first detecting section 54 in the each time breathing may be displayed on the display section 57 sequentially like "49 mmHg" → "45 mmHg" → 47 mmHg → (omitted) →"46 mmHg". Further, when the device body 51 has a sound generator (not illustrated), a prescribed sound may be emitted at a timing when the maximum value of the carbon dioxide concentration detected by the second detecting section 55 is displayed on the display section 57.

When the processing mode of the device body 51 is set in the second processing mode, the maximum value of the carbon dioxide detected by the first detecting section 54 is averaged by the averaging processing section 58, every time the maximum value of the carbon dioxide per one breathing in each time is detected by the first detecting section 54, and the result is displayed on the display section 57. In this case, the maximum value of the carbon dioxide concentration displayed on the display section 57 is updated in real time in accordance with an increase of the respiratory rate of the examinee. Therefore, the carbon dioxide concentration in the exhaled breath can be continuously monitored.

### <6. Effect of the embodiment>

According to this embodiment, one or a plurality of effects shown below are obtained.
(a) In the breath test system 100 of this embodiment, when the examinee performs breathing with the mouthpiece 5 of the breath test device 1 held in the mouth, the maximum carbon dioxide concentration can be displayed on the display section 57, out of the carbon dioxide concentrations in the exhaled breath measured by the measuring section 52 during N-times (six in this embodiment) breathings of the examinee. Thus, the carbon dioxide concentration indicating a higher correlation with respect to the carbon dioxide concentration in the artery, can be displayed. The reason is as follows.
   First, when the examinee has no respiratory disease, a breathing manner is moderately deep, thus showing a stable tendency, and therefore there is a small variation in the output waveform of the carbon dioxide concentration. Accordingly, a difference becomes small between the value obtained by averaging the maximum value of the carbon dioxide concentration detected by the first detecting section 54 while the examinee performs multiple numbers of times of breathing, and the maximum value of the carbon dioxide concentration detected by the second detecting section 55. In contrast, when the examinee is a COPD patient, a breathing manner is shallow, thus showing a non-stable tendency. Therefore the variation is easily generated in the output waveform of the carbon dioxide concentration. Accordingly, the difference becomes relatively large between the value obtained by averaging the maximum value of the carbon dioxide concentration detected by the first detecting section 54 while the examinee performs multiple numbers of times of breathing, and the maximum value of the carbon dioxide concentration detected by the second detecting section 55. Then, from a viewpoint of a correlation with the carbon dioxide concentration in the artery, the maximum value of the carbon dioxide concentration detected by the second detecting section 55 has a higher correlation (similarity), compared to the averaged value described above. Accordingly, by displaying the maximum value of the carbon dioxide concentration detected by the second detecting section 55 on the display section 57, the carbon dioxide concentration showing a higher correlation with respect to the carbon dioxide concentration in the artery, can be displayed as a detection result. Thus, even if the carbon dioxide concentration in the artery is not measured, diagnosis of COPD can be correctly performed. However, the breath test system 100 of this embodiment is not based on a premise that measurement of the carbon dioxide concentration in the artery is not performed. In order to confirm the diagnosis of COPD, the measurement of the carbon dioxide concentration in the artery may be performed separately as needed.
(b) Further, after strenuous efforts by the inventors of the present invention, it is found that even in a case that the examinee is the COPD patient, at least one relatively deep breathing is included in the six breathings, and the maximum value of the carbon dioxide concentration included in the exhaled breath at this time has a strong correlation with the carbon dioxide concentration in the artery. Therefore, it is suitable to set the value of N to six or around six. Thus, the measurement result of the carbon dioxide concentration having a strong correlation with the carbon dioxide concentration in the artery, can be displayed on the display section 57 in a shorter test time (respiratory rate).
(c) The breath test device 1 of this embodiment employs a configuration in which the airway member 3 and the gas sensor 4 are integrally constituted, and the mouthpiece 5 is connected to the airway member 3 so as to communicate with the flow path 30. Accordingly, in the breath test, the concentration of the carbon dioxide contained in the exhaled breath can be measured by the gas sensor 4 only by naturally breathing with the mouthpiece 5 held between the teeth of the examinee. As a result, diagnosis of COPD can be correctly and easily performed in routine checkups. Further, the breath test device 1 can be employed for the routine checkups targeting a large number of people, thus greatly contributing to early detection and early treatment of COPD.
(d) In the breath test device 1 of this embodiment, the mouthpiece 5 is constituted attachably and detachably to/from the airway member 3. Therefore, for example, an unnecessary damage, etc., of the mouthpiece 5 can be avoided by storing the mouthpiece 5 in a state of being detached from the airway member 3 while the breath test is not performed.
(e) In the breath test device 1 of this embodiment, the mouthpiece 5 is disposable. Therefore, when the breath test is performed in routine checkups, the mouthpiece 5 can be used by exchanging it with a new one for each examinee. Accordingly, the breath test can be hygienically performed without taking a trouble of sterilizing the mouthpiece 5.
(f) In the breath test device 1 of this embodiment, the mouthpiece 5 is formed into a straw shape. Therefore, when the mouthpiece 5 is disposable, a manufacturing cost of the mouthpiece 5 can be suppressed to significantly low. Accordingly, when the COPD diagnosis by the breath test is added to diagnosis items of the routine checkups, a cost burden can be reduced. Therefore, in the routine checkups targeting the large number of people, introduction of the COPD diagnosis by the breath test can be encouraged.
(g) In the breath tests device 1 of this embodiment, the airway member 3 is integrally made of PET. Accordingly, a state (such as cleanliness) of the flow path 30 formed by the airway member 3, can be visually confirmed from outside of the airway member 3. Further, the infrared ray is easily transmitted through the detection windows 24a and 24b of the airway member 3, and therefore measurement accuracy of the gas concentration can be increased.
(h) In the breath test device 1 of this embodiment, the airway member 3 has a dividable half-split structure by a pair of airway halves 3a and 3b. Accordingly, for example, when the flow path 30 of the airway member 3 is dirty, the flow path 30 (long grooves 27a and 27b) can be substantially exposed to outside by dividing the pair of airway halves 3a and 3b. Therefore, when a stain is generated on the flow path 30 due to repeated use of the breath test device 1, the stain can be easily and surely removed. Accordingly, the breath test device 1 excellent in maintenance property can be realized.
(i) In the breath test device 1 of this embodiment, recess portions 29a and 29b are formed in a recessed state toward the flow path 30, on the side opposite to the side facing the flow path 30 which is the formation site of the detection windows 24a and 24b of the airway member 3, and the thickness of the portion of the detection windows 24a and 24b is smaller than the other portion due to a dent of the recess portions 29a and 29b. Accordingly, the surface of the flow path 30 can be formed as a smooth continuous surface at the formation site of the detection windows 24a and 24b, while sufficiently increasing a transmittance of the infrared ray through the detection windows 24a and 24b. Therefore, the exhaled breath and the inhaled breath of the examinee can be circulated without being disturbed at the formation site of the detection windows 24a and 24b.
(j) In the breath test device 1 of this embodiment, the case 2 has a dividable half-split structure by a pair of case halves 2a and 2b. Accordingly, the inside of the case 2 can be opened by dividing the pair of case halves 2a and 2b. Therefore, the maintenance (including exchanging) of the airway member 3 and the gas sensor 4 housed in the case 2 can be facilitated.

### <7. Modified example, etc.>

A technical range of the present invention is not limited to the abovementioned embodiment, and includes various modifications or improvements in a range of deriving a specific effect obtained by features of the invention and a combination of them.

For example, in the abovementioned embodiment, to integrally constituting the airway member 3 and the gas sensor 4, the gas sensor 4 is mounted on the airway member 3 using the sensor substrates 28a and 28b. However, the present invention is not limited thereto, and the airway member 3 and the gas sensor 4 may be integrally constituted by mounting the gas sensor 4 on the case 2 in which the airway member 3 is housed, using the sensor substrates 28a and 28b.

Further, in the abovementioned embodiment, the breath test device of the present invention is configured so that the airway member 3 is housed in the case 2, and the cable 6 is connected to the case 2. However, the breath test device of the present invention may be configured without the case 2 or may be configured without the cable 6.

Further, in the abovementioned embodiment, exchange of signals or power between the breath test device 1 and the device body 51, is performed via the cable 6, but may be performed by wireless communication or wireless power supply instead.

Further, in the abovementioned embodiment, one of the maximum value of the carbon dioxide concentration extracted by the extracting section 60, and the maximum value of the carbon dioxide concentration averaged by the averaging processing section 58, is displayed on the display section 57 in accordance with the processing mode (the first processing mode or the second processing mode) of the device body 51 performed by the mode switching section 59. However, the present invention is not limited thereto, and may employ a configuration in which both of the maximum values are simultaneously (together) displayed in the screen of the display section 57.

Further, the breath test device of the present invention may be applied in a case when EtCO₂ is measured targeting not only the patients in routine checkups but also the patients who visit to a medical facility for diagnosis or treatment of COPD, or the patients in home care.

### Description of Signs and Numerals

- 1: Breath test device
- 2: Case
- 3: Airway member (flow path forming member)
- 4: Gas sensor
- 5: Mouthpiece
- 51: Device body
- 52: Measuring section
- 53: Counting section
- 54: Detecting section
- 55: Detecting section
- 57: Display section
- 60: Extracting section
- 100: Breath test system

## Claims

1. A breath test system, comprising:
a breath test device;
a device body communicably connected to the breath test device,
the breath test device comprising:
a flow path forming member having a flow path for flowing a respiratory gas of an examinee;
a gas sensor having a light emitting section that emits an infrared ray to the flow path, and a light receiving section that receives an infrared ray emitted by the light emitting section; and
a mouthpiece connected to the flow path forming member in a state of communicating with the flow path,
the device body comprising:
a measuring section that measures a carbon dioxide concentration contained in an exhaled breath of an examinee, based on an electric signal outputted from the gas sensor;
an extracting section that extracts a maximum value of the carbon dioxide concentration from a measurement result of the measuring section obtained in a period of N-times (N is an integer of 2 or more) breathing of the examinee; and
a display section for displaying a maximum value of the carbon dioxide concentration extracted by the extracting section.

2. The breath test system according to claim 1, wherein the extracting section comprises:
a counting section that counts the number of breaths; and
a first detecting section that detects a maximum value of a carbon dioxide concentration per one breath of each time, based on a count result of the counting section; and
a second detecting section that detects a maximum value of a carbon dioxide concentration in a respiratory rate period of the N-times, by comparing maximum values of a carbon dioxide concentration detected by the detecting section in each time respiration, when the number of times counted by the counting section reaches the N-times,
wherein the maximum value of the carbon dioxide concentration detected by the second detecting section is displayed on the display section.

3. The breath test system according to claim 1 or 2, wherein the N is 3 or more and 10 or less.
